# EUROPEAN PATENT SPECIFICATION

(11) **EP 2 449 086 B1**
(45) Date of publication and mention of the grant of the patent: **10.08.2016**
(21) Application number: 10794409.2
(22) Date of filing: 30.06.2010
(51) Int. Cl.: C12M 1/113, C12M 1/06, C12M 1/02, B01F 7/00, B01F 5/06, B01F 7/10, C02F 11/04, C12M 1/107

(54) **A DEVICE FOR THE PRODUCTION OF BIOGAS FROM ORGANIC WASTE**
VORRICHTUNG ZUR HERSTELLUNG VON BIOGAS AUS ORGANISCHEN ABFÄLLEN
DISPOSITIF POUR PRODUIRE DU BIOGAZ À PARTIR DE DÉCHETS ORGANIQUES

(30) Priority: 02.07.2009 NO 20092495
(43) Date of publication of application: 09.05.2012
(73) Proprietor: Andersen, Uno, 1400 Ski (NO)
(72) Inventor: Andersen, Uno, 1400 Ski (NO)
(74) Representative: Onsagers AS
(86) International application number: PCT/NO2010/000253
(87) International publication number: WO 2011/002303

(56) References cited:
- WO-A1-84/00378
- AU-A1- 2006 202 894
- DE-A1-102005 057 979
- GB-A- 1 317 836
- GB-A- 2 037 731
- JP-A- 2006 255 626
- US-A- 4 514 297

## Description

The invention relates to a fermentation plant for biological degradation of organic material and associated production of biogas. More specifically, the invention relates to a device for biological degradation of organic material and for producing biogas from said degradation, as indicated in the preamble to claim 1.

### Background to the invention

Fermentation plants, also referred to as biogas plants and bioreactors, where biological waste and organic waste from animals and humans are converted to gas by means of anaerobic biological processes, and the waste is broken down to a useful compost mass, are well known. The gas formed contains large amounts of methane (CH₄) as well as e.g. CO₂ and hydrogen sulphide (H₂S), which can be burned to provide heat and power or it is used as fuel in cars and other means of transport.

The fermentation process and the degradation of the biological material take place in a closed container in order to prevent oxygen being supplied from the surrounding air and to enable the biogas produced to be collected in controlled forms.

The degradation of the biological material is time-consuming, requiring a residence time for the material of up to several days in the container. Furthermore, known plants often contain one or more agitating devices and/or circulation pumps.

Biogas plants are often large, complex and capital-demanding, resulting in a high threshold for building such plants. Many local authorities therefore elect to transport the organic waste to far-off plants, which is expensive and energy-demanding, or they do not consider source separation of the waste to be worth while at all, with the result that large amounts of potential energy are lost.

Current indicators regarding increased prices for chemical fertilizer mean that the agricultural industry will go in more for the use of organic fertilizer. This demands more and smaller biogas plants.

From Denmark, amongst other places, a bioreactor is known which converts organic material in the course of 4-5 days and has a relatively high gas production. Bacteria contribute in breaking down the organic material and producing methane gas. This known reactor is divided into chambers with a series of parallel walls, between which the organic mass has to flow. Bacteria become attached to the walls of the chamber. When the chamber has many walls, a large surface is obtained. This provides the basis for a high production of bacteria which participate in converting organic material. Cell division in the culture releases bacteria which intermix with the organic mass and help to convert it.

Bacteria are also utilised for degradation in biological purification processes for sewage in purification plants. After the sedimentation process the sludge water contains a great deal of nutrition

By means of a biological process the sludge water is introduced into a vessel containing open plastic structures; a biological filter. Bacteria will become attached to these when the sludge water runs through, and they are instrumental in breaking down the nutrients. The plastic structures have a given surface. The through-flow rate in the vessel is calculated according to square metre surface of the total amount of plastic structures.

Known bioreactors are dependent on the biomass being heated up, usually by means of heating elements in the tank. The mass must be well stirred in order to maintain a uniform temperature. Several agitators are therefore often mounted in the known bioreactors, or pumps are used to circulate the organic mass. The mass is pumped through a heat exchanger on the way back to the tank. Varying temperature in the biomass gives reduced conversion and gas production. The residence time for organic mass in such reactors may be between 14 and 21 days. Agitation in the tanks will mix bacteria and the various stages the biomass has to undergo during the degradation processes. This may involve longer residence time in the tank before the biomass is converted.

GB2037731 discloses a reactor for anaerobic decomposition comprising a cylindrical vessel with a rotating shaft and rotating partitions with openings. The reactor comprises a return pipeline for returning settlements to an earlier section of the reactor, whereby bacteria are also returned and mixed.

JP2006-255626 discloses a cylindrical shaped digester with an axis and two rotating support plates supporting string like agitation members arranged there between.

De102005057979 discloses a biogas fermenter with a longitudinal container comprising a number of rotating elements arranged on the same number of shafts arranged in parallel perpendicular to the direction of the container.

GB1317836 discloses a fermenter. The rotating discs mounted on an axis comprise openings.

US4514297 discloses a digester for producing methane gas comprising a vessel with a rotating shaft with round discs comprising blades connected thereto.

WO84/00378 discloses a biogas production device comprising a conveyor screw with openings for transporting the organic substrate through the device.

Tanks in biogas plants must be kept clean inside. Agitation mechanisms and heating tubes in the tank also have to be cleaned and sediment in the tank has to be removed. The biomass in large tanks of known type can have problems with it separating into two phases; a top phase and a bottom phase. The formation of foam may cause problems in the gas that is developed. The bottom phase will contain the heavier sediments, while the top phase contains the lighter sediments which may cause problems for plants using pumps for agitation and heating of the biomass. The thinly liquid top phase may form channels through the bottom phase and circulate through the tank and the pumping plant. The heavy sediments in the bottom phase will then fail to circulate, resulting in major problems for the reactor. It is important to have a homogeneous mass in order for the reactor to function optimally. These reactors have a dry solids content of between 5 and 15% in the biomass. The known plants all have to be stopped for periods for cleaning.

It is also important to have good control of the temperature in order to achieve the best possible result. Known bioreactors often consist of a large tank, where the organic mass is circulated through a heat exchanger. The resulting agitation process will also mix the bacteria in the organic mass, but it will also mix the bacteria processes. This may result in reduced conversion and less gas production, which is considered a disadvantage.

There is therefore a need for a plant which solves one or more of the problems encountered in the prior art.

### Summary of the invention

The invention is specified in the independent claim, while the dependent claims specify other characteristics of the invention.

A device is provided for biological degradation of organic material and for producing biogas from said degradation, comprising a closed container (4) with a filling opening (12) for the organic material and discharge openings (5, 18) for the degradation products and one or more rotary elements (26) mounted on a shaft (24) and arranged for rotation in the container, characterised in that each rotary element (26) comprises at least one plate-shaped cylinder body (28; 28a-c) and a moving device (30) for moving the organic material from a first side (30a) of the moving device to a second side (30b) of the moving device, and the cylinder body (28; 28a-c) is connected to the moving device's (30) second side (30b) and protruding therefrom, concentrically about the shaft (24).

In an embodiment each rotary element (30) comprises a plurality of said cylinder bodies (28a-c) with different diameters and connected to the moving device's (30) second side (30b) and protruding therefrom, concentrically about the shaft (24).

In an embodiment the moving device (30) comprises a disc-shaped element (30) arranged substantially perpendicularly to the shaft (24) and with a plurality of through-going openings (32) from the disc's first side (30a) to the disc's second side (30b).

In an embodiment at each opening (30) on the disc-shaped element's first side (30a) there is provided a blade element (34; 34', 34") protruding from the said first side.

In an embodiment the cross sectional profile of the openings in the rotary element's axial plane describes an angle α with the shaft (24) which is different from 0, preferably larger than 0, more preferably equal to 45°.

In an embodiment the container (4) is provided with an external heating jacket (36), whereby the temperature in the organic material can be regulated.

The object of the invention is to provide a compact and independent plant, which is flexible in installation and use, which can handle high proportions of dry solids and which requires a shorter residence time than the known plants.

The bioreactor according to the invention contains large surfaces to which the bacteria can become attached, and the various bacteria will be stationary and stable in the reactor's axial direction. Rotation and feed input can be adjusted according to requirements.

The plant according to the invention can be run with a high dry solids content, with the result that the organic final product will be easier to handle. While the known plants operate with a dry solids content of 5-15% and consequently are dependent on large quantities of water, the plant according to the invention can operate with a dry solids content of 40-50%.

The conversion of the organic mass takes place in several stages. The first stage is hydrolysis. Several different bacteria break down complex compounds and precipitate enzymes which new bacteria use for degradation of cellulose. The hydrolysed products are absorbed by fermentative bacteria which help to form methane.

When the through-flow rate is correct, the various stages will be spread over the chambers in the bioreactor. In each chamber a new process in the degradation of the organic mass takes place. The bacteria remain stationary and the bacteria flora in the tank can be controlled by temperature and the rotational speed of the wings.

The device according to the invention produces gas from the degradation of the organic mass. In this process the problem of foam production will also be reduced, since the wings' rotation will pull the foam back down into the organic mass.

The apertures and the blades in each disc feed the organic mass in a screw movement through the disc, where the mass is allowed to "rest" until it is fed through the next disc.

### Brief decription of the figures

These and other characteristics of the invention will be further explained in the following description of an embodiment, presented as a non-limiting example, with reference to the accompanying drawings, in which:
Figure 1 is a schematic diagram, viewed from the side, of the device according to the invention mounted in a container;
Figure 2 is a schematic diagram of the internal rotary elements in the device according to the invention, where a part of the tank's wall has been removed;
Figure 3a illustrates an embodiment of the rotary element according to the invention, viewed from in front;
Figure 3b illustrates the rotary element depicted in figure 3a, viewed from the side;
Figure 3c illustrates the rotary element depicted in figures 3a and 3b, viewed from the rear;
Figure 4 is a sectional drawing of the rotary element illustrated in figure 3b, along intersecting line A-A illustrated in figure 3a.
Figure 5 is an enlarged section of a portion of figure 4;
Figure 6 is a schematic diagram illustrating an alternative embodiment of the rotary element, viewed from the rear;
Figures 7a and 7b are schematic diagrams illustrating alternative embodiments of apertures and blades;
Figure 8 is a schematic diagram illustrating the tank viewed from the side, partly intersected in order to show a temperature-regulating system; and
Figure 9 illustrates the device depicted in figure 8, viewed from one end.

### Description of an embodiment of the invention

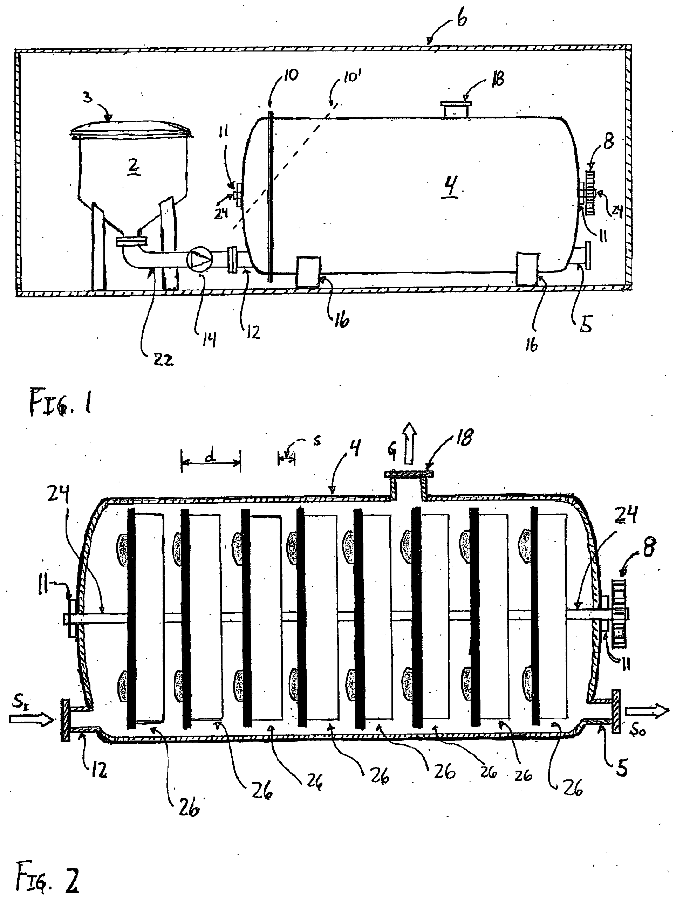

Figure 1 illustrates the device according to the invention mounted together with a pre-treatment unit 2 in a container 6. The pre-treatment unit 2, which may, e.g., be a mill or the like, comprises a filling cap 3 for organic material and a supply pipe 22 which is connected via a pump 14 to an inlet 12 on the container 4. In the pre-treatment unit 2 the organic material is chopped and/or ground and liquid is added in a greater or lesser degree if necessary. The pre-treatment unit is of a known type with the purpose of producing an organic mass which can be fed into the bioreactor.

The bioreactor in the form of a container 4, illustrated here as a cylindrical tank, is supported by bases 16 and has a dividing flange 10, thereby enabling the tank to be opened, e.g. for insertion and removal of components inside the tank. The dotted line 10' indicates an alternative dividing flange.

The container or tank 4 is further provided with an outlet 5 for treated organic mass and an outlet 18 for discharge of gas. Essential connection pipes, pumps and valves connected to the outlets for further treatment of the organic mass are not shown since these units are known in the prior art.

Figure 1 also shows that the tank 4 has a through-going shaft 24, rotatably supported at each end on suitable bearings 11, and drive gear 8 for connection to external motors or the like (not shown), for rotation of the shaft.

We now refer to figure 2, where a part of the tank's 4 wall has been removed. The tank contains a number of rotary elements 26, which are rotatably mounted on the shaft 24, to enable them to rotate when the drive gear 8 is in operation. It may be necessary to have several bearings inside the tank for the shaft 24, but only the two end bearings 11 are shown here.

The rotary elements 26 are mounted on the shaft at a distance s apart, i.e. from the rear end of one rotary element to the front of the following rotary element. The distance between adjacent rotary elements' front sides is indicated by reference letter *d.* The distances s and *d* are dimensioned according to the requirements of the plant concerned.

The arrows marked S_{I} and S_{O} indicate the direction of flow for the organic mass, in and out of the tank respectively, and the arrow marked G indicates the direction of flow for the produced biogas. Thus when terms such as "in front", "behind", "front", "back", etc. are used in the following, this is related to the organic mass's direction of flow through the tank. For example "behind" is downstream of "in front".

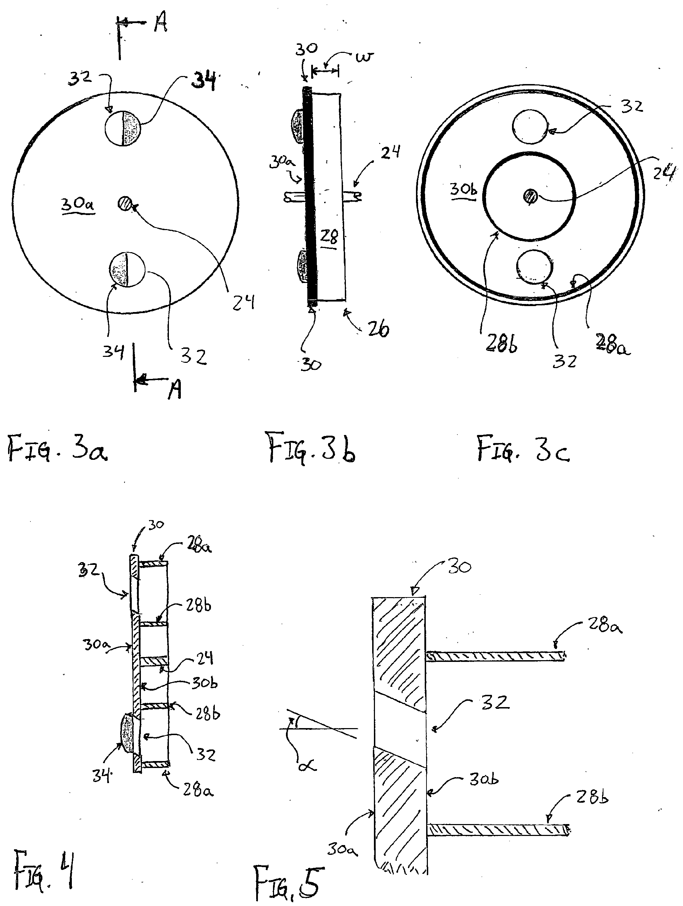

The construction and design of the rotary element 26 will now be described, firstly with reference to figures 3a-c.

The rotary element 26 comprises a circular disc 30 provided with a number of through-going apertures 32, i.e. openings between the disc's 30 front 30a and back 30b. Each aperture 32 is advantageously equipped with a blade 34 which, when the rotary element 26 is rotated, will engage with the organic mass and feed it through the aperture. The disc, with the apertures 32 and the blades 34, therefore acts as a mass moving unit for the organic mass.

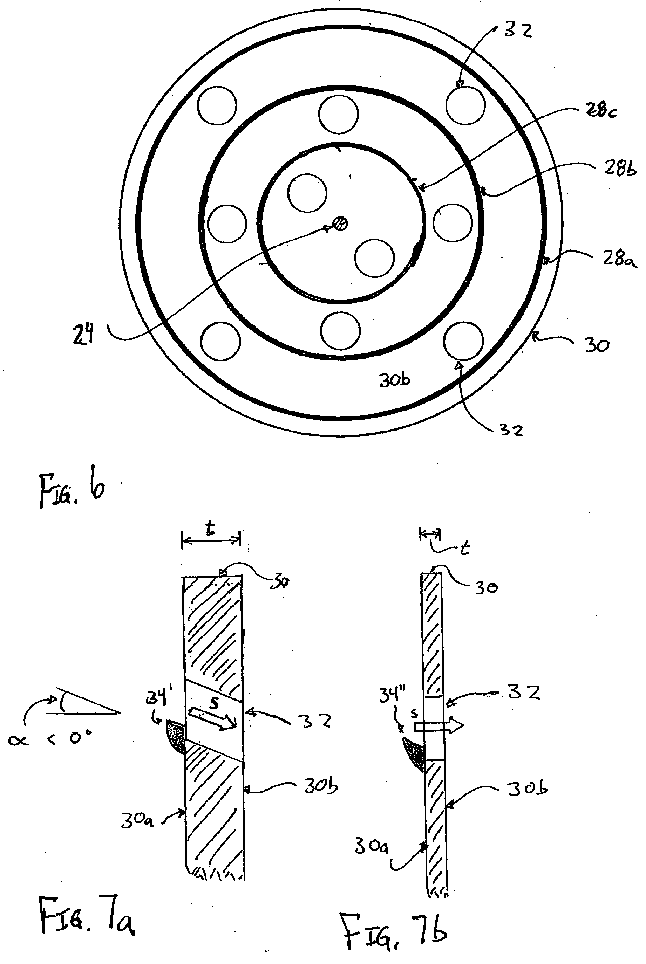

Figure 4, which is a sectional drawing of the rotary element, shows that the apertures 32 are at an angle relative to the shaft 24. Figure 5, which is an enlarged section of an upper part of figure 4, shows that the aperture extends through the disc 30 at an angle α > 0. Figures 7a and 7b illustrate alternative aperture profiles and designs of the blades 34'; 34", as well as illustrating that the disc's thickness *t* may be varied in the different embodiments.

As illustrated in figures 3c, 4 and 5, two cylindrical or barrel-shaped elements 28a, 28b are attached to the back 30b of the disc. The cylindrical elements, also referred to as "wings" in the following, are arranged concentrically about the shaft 24, and the apertures 32 are arranged between the wings 28a, b. It should be understood that the number of wings and the number of apertures between the wings can be adapted to suit requirements, e.g. the size of the tank, the nature of the organic mass and the desired through-flow rate. Similarly, the wings' axial dimension w is calculated according to requirements for the plant concerned, preferably together with the parameters *s* and *d* as described above. Thus figure 6 illustrates an alternative embodiment with three wings 28a, b, c concentric about the shaft 24, and a larger number of apertures 32 between the wings.

The bioreactor's core is therefore one or more rotary elements 26, designed as described above and mounted on the shaft 24.

When the rotary element is rotated, organic mass will be passed through the apertures in the disc and enter the cavity formed by the back 30b of the disc and the associated wing.

The rotating disc 30 and the wings 28a-c mounted thereon form a large surface which is exposed to the organic mass. On this surface bacteria will build up which are instrumental in converting the organic mass, thereby producing biogas amongst other things.

When the bioreactor is in use, the organic mass is pumped into the reactor from the pre-treatment mill 2. As mentioned above, the size of the apertures 32 in the discs 30, together with the discs' rotational velocity, will help to determine the rate at which the mass is fed into the reactor. In this way the organic mass will be pressed from disc to disc through the tank 4. The process will thereby be highly controlled and the bacteria cultures will convert the organic mass.

During use, the input of organic mass is regulated so that it preferably occupies between % and % of the tank's volume. The discs 30 will rotate relatively slowly through the organic mass that will be in the tank. The cylindrical wings 28a-c and the associated disc provide a relatively large surface which is exposed to the mass.

When the discs 30 and the wings 28a-c are in contact with the organic material, bacteria will attach themselves to the surfaces of these elements. The bacteria will thereby become stationary in the reactor's axial direction, and with good access to new organic material, they will increase in number.

The rotary element 26 will rotate relatively slowly. The bacteria culture growing on the surface of the disc and the wings will thereby make good contact with the organic material. Some bacteria will fall and be mixed into the material, and new ones will develop on the disc. Detached bacteria will intermix with the organic mass, providing a faster conversion.

An anaerobic degradation takes place in several phases. These phases will occur in a specific order in the device according to the invention. The different processes will be naturally distributed on the discs in the bioreactor. The rotary elements 26 will act as physical separators which will prevent the processes from becoming mixed.

When the organic mass enters the space defined by the back 30b of the disc and the wings 28a-c, it is mixed with bacteria. The mass will remain between the wings for a period before being fed into the apertures 32 in the next (downstream) disc. In the space behind this next disc, there may be another bacteria culture which continues to break down other substances in the same organic mass, or it may continue the degradation of what has been started.

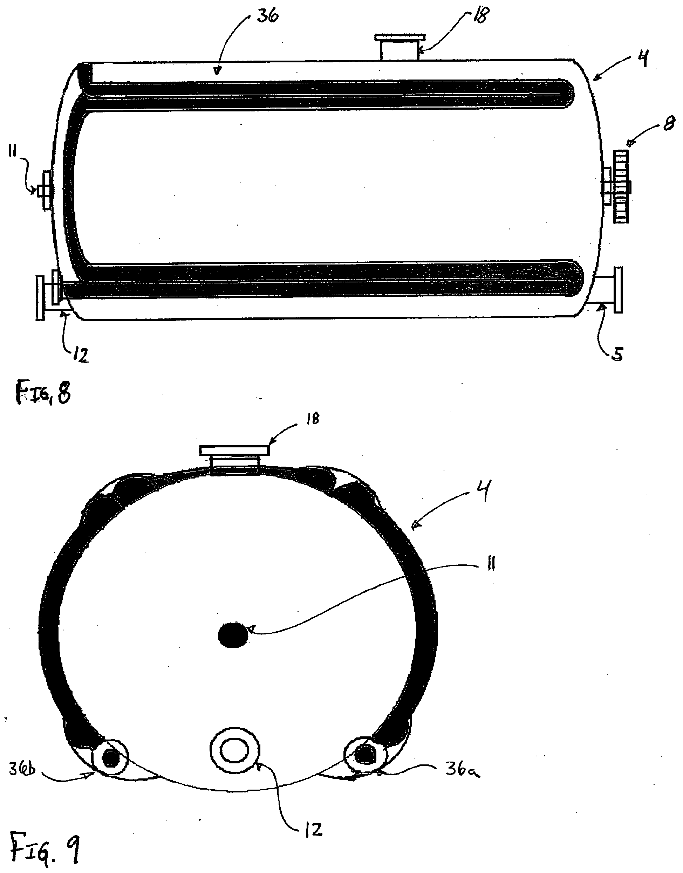

The rotary elements' discs with the associated cylindrical wings thus form a large surface on which the bacteria can grow. Large surfaces provide good opportunities for bacterial growth, but it is also important to have good control of the temperature in order to achieve the best possible result. This may be controlled, for example, by an insulated heating jacket on the outside of the tank, as schematically illustrated in figures 8 and 9, viewed from the side and from one end respectively. A pipe system 36 extends along the tank's wall and has an inlet 36a and an outlet 36b for connection to essential pumps, heat exchangers etc. of a known type. The object of the heating jacket is to ensure a stable temperature in the reactor, and not to heat up the contents of the tank. In an embodiment the heating jacket may be in the form of imbedded plastic channels 36 on the outside of the tank, where water or another thermal medium can circulate. On the outside of the channels an insulating layer (not shown) may be mounted in order to prevent heat loss.

In one embodiment the temperature in the heating jacket may be around 55°C. Since the composition of the bacteria culture and the resulting gas mixture are temperature-sensitive, the heating jacket may also be used for controlling the temperature inside the tank, in order thereby to produce as much as possible of the desired type of gas.

As mentioned above, the apertures 32 in the discs 30 work in such a manner that the organic mass is passed to the space defined by the back 30b of the disc and the associated cylindrical wings 28a-c. As mentioned and illustrated in figures 4, 5 and 7a, the cross section of the apertures 32 is advantageously at an angle relative to the shaft 24. It is preferable that the angle α = 45°. In order to facilitate the introduction of the organic mass through the disc, a protruding cup or blade 34 may be mounted on the apertures on the front 30a of the disc. Figures 7a, b illustrate alternative embodiments of the blades 34'; 34".

When the disc rotates in the same direction as the blades' opening, the blades help to guide the mass into and through the apertures, illustrated by the arrows S in figures 7a and 7b. As mentioned, the position and size of the apertures must be calculated and located in such a manner that the correct amount of organic mass is guided in between the cylindrical wings. The volume defined by the wings must therefore be calculated first so that they are in proportion to one another.

### Example - compact plant

A standard (40 foot) container 6 can hold a tank 4 with a diameter of 2.10m and a length of 10m, i.e. a volume of approximately 35m³.

The unit is thermophilic; the temperature in the tank 4 is kept at a uniform level, preferably between 55°C and 57°C by means of the heating jacket 36. The heating jacket 36 transports hot water and comprises pipes on the top and bottom of the tank 4 (cf. figures 8 and 9). The temperature in the tank can be regulated and controlled by this heating jacket. The space between the tank's heating jacket and the container is preferably insulated in order to avoid heat loss.

Residence time in the tank for the organic material is about 5-6 days.

The rotary elements 26 in the tank 4 rotate through the organic mass. On each rotary element 26 the disc 30 and the associated wings 28; 28a-c together form a large surface which is exposed to the organic mass. This surface will be covered by bacteria, which will contribute to the degradation of the organic material. When the rotary elements 26 rotate, they will provide the basis for more bacterial growth, and bacteria will intermix with the organic material, speeding up the degradation. It is a known fact that anaerobic degradation of organic mass is fundamental to the production of methane. By developing a good bacteria culture and efficient admixture of bacteria in the organic mass, a good basis will be achieved for a high production of methane gas.

### Design data:

- Tank volume:: 35m³
- Disc diameter:: 1.9m
- Number of wings per disc:: 5

Radial distance between the wings:0.2m

This gives the following areas which are exposed to organic mass:

| **Component** | **Diameter (m)** | **Exposed surface (m²)** |
|---|---|---|
| Feed disc | 1.9 | 5.67 |
| Wing 1 | 1.9 | 11.93 |
| Wing 2 | 1.5 | 9.42 |
| Wing 3 | 1.1 | 6.91 |
| Wing 4 | 0.7 | 4.40 |
| Wing 5 | 0.3 | 1.88 |
| **Total per rotary element** | | **40.21** |

One rotary element rotating slowly through the organic mass will therefore obtain a total surface of approximately 40m² to which bacteria can become attached.

A tank which is 10 metres long will contain ten rotating discs with wings, giving a total surface of 400m² which will be covered by different bacteria.

Input of organic mass is calculated at 5m³ per 24 hours. This is a small volume compared to other biogas plants, but by adding little water and having a high dry solids content, this plant can cover the needs of 8,000 to 10,000 households in a municipality.

With a regular feed input to the tank, 5m³ will be stirred and come into contact with bacteria growing on 80m² distributed on two discs with wings.

The tank should only be filled ¾ full with organic mass. This means that there will be an air space which will be filled with gas from the degradation of the organic mass. In this process foam will also be able to build up, but the wings' rotation will pull the foam back down into the organic mass. The wings' rotation will also create currents and movements in the organic mass. When the tank is only % full, the organic mass will not be able to rotate together with the wings. The rotation will be broken up and a turbulent current will be created on the side at which the wings are lifted up from the organic mass.

Organic mass will thereby be stirred and mixed with bacteria which contribute to degradation and conversion.

The degradation is divided into two main groups: hydrolysis and fermentation.

The hydrolysis breaks down organic components such as carbohydrates, protein and fat. Different bacteria contribute to this degradation. A high cellulose content can reduce the rate of degradation somewhat.

The hydrolysed products are further broken down by fermentative bacteria. In a well-functioning anaerobic environment, many of the organic compounds will be more rapidly converted by the fermentative bacteria to methane and hydrogen compounds.

The degradation takes place in a specific order, and the discs' rotation and input of organic mass provide very good control of bacteria and conversion.

There are many different biogas plants. One of the advantages of the plant according to the invention is that it can be run with a high dry solids content. The organic mass may resemble a thick porridge, making the organic final product easier to handle. The high temperature can be exploited when the mass has to be dried further and run through a pellets plant. The organic mass will be easy to handle as pellets.

Several plants according to the invention may be coupled together in series if so desired.

## Claims

1. A device for biological degradation of organic material and for producing biogas from said degradation, comprising a closed container (4) with a filling opening (12) for the organic material and discharge openings (5, 18) for the degradation products and one or more rotary elements (26) mounted on a shaft (24) and arranged for rotation in the container, **characterised in that** each rotary element (26) comprises at least one cylinder body (28; 28a-c) and a disc-shaped element (30) arranged perpendicularly to the shaft (24) and with a plurality of through-going openings (32) from a first side (30a) of the disc-shaped element (30) to a second side (30b) of the disc-shaped element (30) for moving the organic material from the first side (30a) to the second side (30b), and the cylinder body (28; 28a-c) is connected to the disc-shaped element's (30) second side (30b) and protruding therefrom, concentrically about the shaft (24).

2. A device according to claim 1, where each rotary element (26) comprises a plurality of said cylinder bodies (28a-c) with different diameters and connected to the disc-shaped element's (30) second side (30b) and protruding therefrom, concentrically about the shaft (24).

3. A device according to claim 1, where at each opening (32) on the disc-shaped element's first side (30a) there is provided a blade element (34; 34', 34") protruding from the said first side.

4. A device according to claim 1, 2 or 3, where the cross sectional profile of the openings in the rotary element's axial plane describes an angle α with the shaft (24) which is different from 0, preferably larger than 0, more preferably equal to 45°.

5. A device according to any of the preceding claims, where the container (4) is provided with an external heating jacket (36), whereby the temperature in the organic material can be regulated.

## Patentansprüche

1. Vorrichtung zum biologischen Abbauen von organischem Material und zum Erzeugen von Biogas aus dem Abbau, wobei die Vorrichtung einen geschlossenen Behälter (4) mit einer Einfüllöffnung (12) für das organische Material und Ausgabeöffnungen (5, 18) für die Abbauprodukte und ein oder mehrere drehbare Elemente (26), die an einer Welle (24) angebracht sind und für eine Drehung in dem Behälter ausgelegt sind, umfasst, **dadurch gekennzeichnet, dass** jedes drehbare Element (26) wenigstens einen zylinderförmigen Körper (28; 28a-c) und ein scheibenförmiges Element (30), das senkrecht zu der Welle (24) angeordnet ist und mehrere Durchgangsöffnungen (32) von einer ersten Seite (30a) des scheibenförmigen Elements (30) zu einer zweiten Seite (30b) des scheibenförmigen Elements (30) zum Bewegen des organischen Materials von der ersten Seite (30a) zu der zweiten Seite (30b) aufweist, umfasst, und wobei der zylinderförmige Körper (28; 28a-c) mit der zweiten Seite (30b) des scheibenförmigen Elements (30) verbunden ist und davon konzentrisch zu der Welle (24) vorsteht.

2. Vorrichtung nach Anspruch 1, wobei jedes drehbare Element (26) mehrere zylinderförmige Körper (28a-c) mit unterschiedlichen Durchmessern umfasst, die mit der zweiten Seite (30b) des scheibenförmigen Elements (30) verbunden sind und davon konzentrisch zu der Welle (24) vorstehen.

3. Vorrichtung nach Anspruch 1, wobei bei jeder Öffnung (32) auf der ersten Seite (30a) des scheibenförmigen Elements ein Klingenelement (34; 34'; 34") vorgesehen ist, das von der ersten Seite vorsteht.

4. Vorrichtung nach einem der Ansprüche 1, 2 oder 3, wobei das Querschnittsprofil der Öffnungen in der axialen Ebene des drehbaren Elements einen Winkel α mit der Welle (24) einschließt, der sich von 0° unterscheidet, vorzugsweise größer als 0° ist und stärker bevorzugt 45° beträgt.

5. Vorrichtung nach einem der vorhergehenden Ansprüche, wobei der Behälter (4) mit einem äußeren Heizmantel (36) versehen ist, wodurch die Temperatur in dem organischen Material reguliert werden kann.

## Revendications

1. Dispositif de dégradation biologique de matière organique et de production de biogaz à partir de ladite dégradation, comprenant un récipient fermé (4) avec une ouverture de remplissage (12) pour la matière organique et des ouvertures d'évacuation (5, 18) pour les produits de dégradation et un ou plusieurs éléments rotatifs (26) montés sur un arbre (24) et disposés pour la rotation dans le récipient, **caractérisé en ce que** chaque élément rotatif (26) comprend au moins un corps de cylindre (28 ; 28a-c) et un élément en forme de disque (30) agencé perpendiculairement à l'arbre (24) et avec une pluralité d'ouvertures débouchantes (32) d'un premier côté (30a) de l'élément en forme de disque (30) à un second côté (30b) de l'élément en forme de disque (30) pour déplacer la matière organique du premier côté (30a) au second côté (30b), et le corps de cylindre (28 ; 28a-c) est raccordé au second côté (30b) de l'élément en forme de disque (30) et faisant saillie de celui-ci, concentriquement autour de l'arbre (24).

2. Dispositif selon la revendication 1, dans lequel chaque élément rotatif (26) comprend une pluralité desdits corps de cylindre (28a-c) avec différents diamètres et raccordés au second côté (30b) de l'élément en forme de disque (30) et faisant saillie de celui-ci, concentriquement autour de l'arbre (24).

3. Dispositif selon la revendication 1, dans lequel au niveau de chaque ouverture (32) sur le premier côté (30a) de l'élément en forme de disque est fourni un élément lame (34 ; 34', 34") faisant saillie dudit premier côté.

4. Dispositif selon la revendication 1, 2 ou 3, dans lequel le profil en coupe des ouvertures dans le plan axial de l'élément rotatif décrit un angle α avec l'arbre (24) qui est différent de 0, de préférence supérieur à 0, de manière davantage préféré égal à 45°.

5. Dispositif selon l'une quelconque des revendications précédentes, dans lequel le récipient (4) est doté d'une gaine chauffante externe (36), permettant de réguler la température dans la matière organique.
